# EUROPÄISCHE PATENTSCHRIFT

(11) **EP 0 421 371 B1**
(45) Veröffentlichungstag und Bekanntmachung des Hinweises auf die Patenterteilung: **17.08.1994**
(21) Anmeldenummer: 90118908.4
(22) Anmeldetag: 03.10.1990
(51) Int. Cl.: A61K 6/10

(54) **Polyalkylenoxidderivate enthaltende Polyetherabformmassen**
Impression materials containing polyalkyleneoxide derivatives
Matériaux d'impression contenant des dérivés d'oxydes de polyalkylènes

(30) Priorität: 03.10.1989 DE 3932989
(43) Veröffentlichungstag der Anmeldung: 10.04.1991
(73) Patentinhaber: THERA Patent GmbH & Co. KG Gesellschaft für industrielle Schutzrechte, D-82229 Seefeld (DE)
(72) Erfinder: Zahler, Wolf-Dietrich, Dr., W-8031 Hechendorf (DE); Gasser, Oswald, Dr., W-8031 Seefeld (DE); Ellrich, Klaus, Dr., W-8031 Seefeld (DE)
(74) Vertreter: Abitz, Walter, Dr.-Ing.

(56) Entgegenhaltungen:
- EP-A- 0 110 429
- EP-A- 0 279 238
- US-A- 3 453 242
- US-A- 4 167 618

## Beschreibung

Bei der Herstellung von Arbeitsmodellen in der Zahntechnik sowie präzisen Abdrücken und provisorischen Zahnersatzteilen in der Zahnheilkunde werden Silikone, Alginate und Hydrokolloide eingesetzt. So beschreibt beispielsweise die US-PS 4 778 832 Silikonmassen, die hydrophile Silikonöle oder nicht-ionische Tenside enthalten, um die Massen hydrophil zu machen. Als nicht-ionische Tenside kommen unter anderem Polyether und Partialester von Polyalkoholen und Fettsäuren in Betracht.

Für besonders präzise Arbeiten werden Polyetherabformmassen eingesetzt. Solche Abformmassen bestehen in der Regel aus Polyalkylenoxiden, bei welchen die Endgruppen mit polymerisationsfähigen Verbindungen derivatisiert sind. So sind z.B. in den US-PSen 3453242 und 4093555, in der DE-PS 1 544 837 und in der DE-OS 32 46 654 aziridinhaltige Polyalkylenoxide für diesen Einsatzzweck beschrieben. Die aziridinhaltigen Verbindungen werden üblicherweise zusammen mit Füllstoffen, Farbstoffen und weiteren Hilfsstoffen, wie z.B. Weichmachern, verwendet.

Zur Initiierung der Polymerisationsreaktionen sind z.B. aus der US-PS 4167618 Sulfoniumsalze bekannt, die in β-Stellung zum zentralen Schwefelatom eine elektronenanziehende Gruppe sowie ein nicht-nucleophiles Anion enthalten.

Zur Einstellung der Verarbeitungseigenschaften sowie des plastisch/elastischen Verhaltens werden den aziridinhaltigen Verbindungen sowie der Komponente, die den Katalysator enthält, üblicherweise sog. Weichmacher sowie Lösungsvermittler zugesetzt. So sind z.B. hydroxyfunktionelle Polyalkylenoxide, wie die Copolymeren aus Ethylenoxid und Propylenoxid, Phthalate, wie z.B. Dioctylphthalat, Zitrate, wie z.B. Acetyltrialkylzitrat, sowie Toluol und Dibenzyltoluol für diesen Einsatzzweck beschrieben worden.

Der Einsatz solcher Verbindungen ist auch aus anderen Gebieten bekannt. So beschreibt die GB-PS 621 104 die Verwendung von Polyalkylenoxiden und Polyalkylenoxidglykolen als Tenside in Farbdispersionen und dergleichen. Die DE-OS 37 12 646 erläutert die Verwendung von Alkyl-Polyoxyalkylencarboxylverbindungen als wesentliche Bestandteile in hautpflegenden Mitteln und die DE-OS 37 02 178 betrifft den Einsatz von Polyglycerinethern als hautverträgliche Zusätze in kosmetischen und pharmazeutischen Mitteln.

Die Polyetherabformmassen werden üblicherweise in Form von zwei räumlich voneinander getrennten Komponenten bis zur eigentlichen Verwendung aufbewahrt. Durch intensives Mischen entsteht dann die reaktive Abformmasse, die in wenigen Minuten erhärtet. Hierbei enthält eine Komponente den Polyether, z.B. die aziridinhaltige Verbindung und die andere Komponente den Katalysator, z.B. die Sulfoniumsalze. Da in der ersten Komponente relativ hochmolekulare Verbindungen und in der zweiten Komponente relativ niedermolekulare Verbindungen verwendet werden, weicht das Mischungsverhältnis auch bei Zusatz von Füllstoffen und Weichmachern sowie Lösungsvermittlern üblicherweise deutlich von einem Mischungsverhältnis 1:1 ab.

Zur Vermischung der Komponenten stehen dem Zahnarzt neben dem Vermischen mit einem Spatel auf dem Block mechanische Mischhilfen zur Verfügung. Hier sind einerseits Zentrifugenmischer bekannt, andererseits ist bekannt, die Komponenten durch statische Vermischung miteinander homogen zu vermischen. Hierbei werden die beiden Komponenten in Doppelkammerkartuschen aufbewahrt und kurz vor der Anwendung durch eine Mischdüse, in welcher ein statisches Mischelement angebracht ist, hindurchgedrückt. Dabei werden die Komponenten durch die spezielle Form der Mischwendeln miteinander vermischt. In diesem System müssen die Fliesseigenschaften der beiden Komponenten so ähnlich wie möglich sein, ausserdem sollte das chemische Verhalten so ähnlich sein, dass eine augenblickliche Vermischung bei kurzen Kontaktzeiten eintritt.

Zur Einstellung eines Mischverhältnisses bei Polyetherabformmassen, welches geeignet ist, sich mit statischen Mischern vermischen zu lassen, war es nötig, in die Komponente, welche den Katalysator enthält, relativ grosse Mengen an Füllstoffen und Weichmachern und/oder Lösungsvermittlern einzubringen. Mit den bekannten Weichmachern und/oder Lösungsvermittlern war dies bisher nur ungenügend möglich, da zum einen Lagerinstabilitäten, insbesondere Entmischungen, auftraten, oder aber das rheologische Verhalten der beiden Komponenten deutlich voneinander abwich, bzw. sich beide Komponenten chemisch so unterschieden, dass sie sich in einem statischen Mischer nicht homogen vermischen liessen.

Aufgabe der Erfindung ist demnach die Bereitstellung einer Polyetherabformmasse, bei der das rheologische sowie chemische Verhalten der beiden Komponenten aneinander angeglichen ist, so dass eine optimale Vermischung auch in Mischdüsen mit einem statischen Mischelement erreicht wird, und bei der es möglich ist, grosse Mengen an Weichmachern und/oder Lösungsvermittlern sowie Füllstoffen in die Komponenten einzuarbeiten, ohne jeweils die physikalischen Endeigenschaften des erhärteten Materials sowie die Fliesseigenschaften der angemischten Komponenten negativ zu beeinflussen.

Gegenstand der Erfindung ist eine Polyetherabformmasse, enthaltend
(a) Polyether,
(b) Katalysator,
(c) Vermischungsadditiv, und
(d) gegebenenfalls übliche Zusatzstoffe,
wobei die Komponenten (a) und (b) räumlich getrennt voneinander vorliegen, welche dadurch gekennzeichnet ist, dass das Vermischungsadditiv (c) ein Polyalkylenoxidderivat mit einem Molekulargewicht über 300 ist, welches gegenüber den anderen vorliegenden Komponenten chemisch inert ist und insbesondere keine Hydroxylgruppen, Säuregruppen, primäre Aminogruppen, ionische Gruppen, Halogengruppen oder polymerisationsfähige Gruppen enthält.

Unter Polyalkylenoxid wird hierbei ein Homo- oder Copolymer aus einem oder mehreren verschiedenen Alkylenoxiden verstanden. Bei Copolymeren können die Monomeren regelmässig oder unregelmässig alternierend angeordnet sein, es kann jedoch auch zunächst aus einem Alkylenoxid ein Homopolymer hergestellt werden, welches dann mit einem oder mehreren anderen Monomeren (Alkylenoxiden) "gepfropft" wird.

Bevorzugt ist die Verwendung von Polyalkylenoxidderivaten der allgemeinen Formel I:

R-X-[(CH₂)ₙ-(CHR′)-O]ₖ-[(CH₂)ₘ-(CHR˝)-O]ₗ-(CH₂)ₘ-(CHR˝)-X-R (I)

worin bedeuten:
- n =: 1 bis 6, vorzugsweise 1 bis 4, insbesondere 1, wobei n innerhalb der Kette variieren kann,
- m =: 1 bis 6, vorzugsweise 1 bis 4, insbesondere 3, wobei m innerhalb der Kette variieren kann,
- k, l =: 2 bis 500, bevorzugt 4 bis 250, insbesondere 10 bis 200,
- R′, R˝ =: H, Methyl, Ethyl, bevorzugt ist R′ = R˝ = H
- X =: S, O, NH, vorzugsweise O,
- R =: C₁₋₁₈-, vorzugsweise C₁₋₁₂-, insbesondere C₁₋₆-Alkyl, oder Carbonyl-C₁₋₁₇-, vorzugsweise Carbonyl-C₁₋₁₁-, insbesondere Carbonyl-C₁₋₅-Alkyl, oder
einen Rest der allgemeinen Formel II: 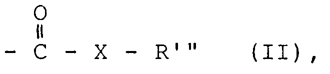
- R‴ =: C₁₋₁₈-, vorzugsweise C₁₋₁₂-, besonders bevorzugt C₁₋₆-Alkyl und/oder -Aryl bedeutet und X die oben angegebene Bedeutung hat,
wobei die mit den Symbolen k und l indizierten Klammerausdrücke regelmässig oder unregelmässig alternierend oder in Blockform angeordnet sein können.

Formel I beschreibt hierbei Verbindungen, enthaltend ein Polyalkylenoxidgrundgerüst, welches den Bauteil zwischen den beiden Gruppierungen X in Formel I bedeutet. Hierbei kann es sich um Homo- oder Copolymerisate von Alkylenoxiden wie Ethylenoxid, Propylenoxid, Tetrahydrofuran, Trioxetan u.ä. handeln, bevorzugt sind die Copolymere aus Ethylenoxid und Tetrahydrofuran. Die Copolymere können hierbei regelmässig wie unregelmässig alternierende Struktur haben. Die verwendeten Homo- bzw. Copolymere haben vorzugsweise mittlere Molekulargewichte zwischen 300 bis 20.000, besonders bevorzugt zwischen 500 bis 10.000. Die Herstellung erfolgt in an sich bekannter Weise durch kationische Polymerisation der Alkylenoxide, beispielsweise mit Katalysatoren des Typs BF₃ bzw. BF₃-Etherat. Hierbei entstehen üblicherweise Homo-bzw. Copolymere mit Hydroxylendgruppen, diese können jedoch in an sich bekannter Weise in SH- bzw. NH₂-Endgruppen überführt werden. Die Gruppe X gibt in Verbindung mit einem Wasserstoffatom die Endgruppe des Polyalkylenoxides an. Diese kann sein eine OH-Gruppe, eine SH-Gruppe oder eine NH₂-Gruppe, welche dann mit Verbindungen RH durch Verätherungs- oder Veresterungsreaktionen sowie durch Reaktion mit Isocyanaten bzw. über Mehrstufenreaktionen, wie die Reaktion mit Phosgen und Aminen, umgesetzt werden. Vorzugsweise sind die Verbindungen der Formel I die Umsetzungsprodukte von Polyalkylenoxiden (mit Hydroxyendgruppen) mit aliphatischen Monocarbonsäuren, bevorzugt sind die Umsetzungsprodukte der hydroxyfunktionellen Polyalkylenoxide mit Ameisen-, Essig-, Propion- und Buttersäure.

Vorzugsweise befindet sich das erfindungsgemäss einzusetzende Polyalkylenoxidderivat in der Komponente, die den Katalysator, vorzugsweise das Sulfoniumsalz, enthält.

Die Polyether (a) enthalten ein Polyalkylenoxid-Grundgerüst, wie es beim Vermischungsadditiv (c) definiert wurde, jedoch mit reaktiven Endgruppen, z.B. Aziridin-Endgruppen, die in der Lage sind, unter Zugabe des Katalysators (b) zu polymerisieren.

Als Polyether (a) werden bevorzugt die aziridinhaltigen Polyalkylenoxidverbindungen eingesetzt, wie sie z.B. in den US-PSen 3453242 und 4093555 beschrieben sind. Als Katalysatoren (b) sind die aus der US-PS 4167618 bekannten Sulfoniumsalze bevorzugt.

Die Menge des Polyethers bzw. der bevorzugten Aziridinverbindung beträgt, berechnet auf die Gesamtmasse beider Komponenten, vorzugsweise 20 bis 80 Gew.-%, besonders bevorzugt 32 bis 60 Gew.-%, und insbesondere 45 bis 55 Gew.-%.

Der Katalysator und insbesondere die bevorzugten Sulfoniumsalze werden vorzugsweise in Mengen von 0,2 bis 24 Gew.-%, besonders bevorzugt 1 bis 18 Gew.-%, insbesondere 5 bis 15 Gew.-%, bezogen auf das Gewicht der Gesamtmasse, eingesetzt.

Die erfindungsgemäss einzusetzenden Polyalkylenoxidderivate werden in Mengen von vorzugsweise 2 bis 60 Gew.-%, besonders bevorzugt 8 bis 50 Gew.-%, und insbesondere 10 bis 40 Gew.-%, bezogen auf die Gesamtmasse, eingesetzt.

Die Polyetherabformmassen können ferner Füllstoffe sowie Thixotropie-Hilfsmittel enthalten, um eine pastenähnliche Konsistenz einzustellen. Geeignte Füllstoffe sind beispielsweise Quarze, CaCO₃, Kieselgure sowie pyrogenes Siliciumdioxid. Es kann vorteilhaft sein, die Füllstoffoberflächen zu behandeln. Geeignete Oberflächenbehandlungsmittel sind Silane, wie z.B. Hexamethyldisilazan.

Weiterhin können ausser den erfindungsgemässen Polyalkylenoxidderivaten weitere Weichmacher und/oder Lösungsmittel, wie sie bereits bekannt sind, eingesetzt werden.

Die Zusatzstoffe (Füllstoffe, Weichmacher, Lösungsmittel etc.) werden zusammen in Mengen von 0 bis 70 Gew.-%, vorzugsweise 10 bis 50 Gew.-%, besonders bevorzugt 20 bis 40 Gew.-%, bezogen auf das Gewicht der Gesamtmasse, eingesetzt.

Die erfindungsgemässen Zusammensetzungen eignen sich insbesondere zur Anwendung in statischen Mischern.

### Herstellungsbeispiel 1

160 g eines Copolymers aus Ethylenoxid und Tetrahydrofuran (im Verhältnis 1:1, Molekulargewicht ca. 3.000) werden mit 80 g Cyclohexan und 130 mg Dibutylzinndilaurat zu einer homogenen klaren Lösung verrührt. Bei 23°C werden 5,8 g Butylisocyanat zugegeben und die Reaktionsmischung 1 Stunde gerührt, wobei die Temperatur auf ca. 30°C ansteigt. Nachdem die Reaktionsmischung wieder Raumtemperatur erreicht hat, wird noch 24 Stunden bei Raumtemperatur weitergerührt, anschliessend wird überschüssiges Isocyanat und Cyclohexan abdestilliert. Man erhält das Polyalkylenoxidbisbutylurethan in quantitativer Ausbeute.

### Herstellungsbeispiel 2

In zu Beispiel 1 analoger Weise wird mit 6 g Cyclohexylisocyanat (statt Butylisocyanat) umgesetzt. Die Wärmetönung erreicht 31°C nach 1 Stunde; es wird 24 Stunden bei Raumtemperatur weitergerührt und wiederum überschüssiges Isocyanat und Lösungsmittel abdestilliert. Das Polyalkylenoxidbiscyclohexylurethan wird in quantitativer Ausbeute erhalten.

### Herstellungsbeispiel 3

600 g eines Copolymers aus THF und Ethylenoxid (im Verhältnis 2:1, Molekulargewicht 6.000) werden in 1,3 l Cyclohexan aufgelöst. Anschliessend werden 35,2 g Buttersäure und 3,5 g Toluolsulfonsäure zugegeben und 20 Stunden unter Rückfluss und gleichzeitiger Wasserabscheidung erhitzt. Anschliessend wird 3 x mit je 200 ml 2nNaOH und 1 x mit 2nH₂SO₄ sowie anschliessend 2 x mit 200 ml H₂O extrahiert. Anschliessend wird über Natriumsulfat getrocknet und restliches Lösungsmittel abdestilliert. Als Ausbeute erhält man 500 g Polyalkylenoxidbisbutyrat.

### Herstellungsbeispiel 4

In zu Beispiel 3 analoger Weise wird mit Acetanhydrid, 19,4 g (statt Buttersäure) umgesetzt und 4 Stunden unter Rückfluss erhitzt. Anschliessend wird, wie in Beispiel 3, 10 Stunden unter gleichzeitiger Wärmeabscheidung weitererhitzt. Die Aufarbeitung erfolgt wie bei Beispiel 3 mittels zweimaliger Extraktion mit wässriger Natronlauge (2n), 1 x Extraktion mit wässriger Schwefelsäure (2n) sowie zweimaliger Extraktion mit H₂O dest. Anschliessend wird wieder mit Natriumsulfat getrocknet und das Lösungsmittel abdestilliert. Man erhält 500 g Polyalkylenoxidbisacetat.

### Anwendungsbeispiel

100 Gewichtsteile eines Polyethers mit Aziridinoendgruppen (durchschnittliches Molgewicht ca. 6.500), dessen Herstellung in der US-PS 3453742 (Beispiel 13) beschrieben ist, werden mit 5 Gewichtsteilen Acetyltributylzitrat und 40 Gewichtsteilen feinem Kieselgur zu einer homogenen Paste verknetet. Die Paste wird im weiteren als Basispaste bezeichnet.

50 Gewichtsteile Acetyltributylzitrat, 50 Gewichtsteile Dibenzyltoluol sowie 40 Gewichtsteile β(S-Lauryl-S-ethylsulfonium)-butyronitril-fluorborat werden unter leichtem Erwärmen solange vermischt, bis eine homogene klare Lösung entsteht. Jeweils 50 Gewichtsteile dieser Lösung werden mit 40 Gewichtsteilen feinem Kieselgur sowie den in der folgenden Tabelle angegebenen Mengen an Vermischungsadditiv zu einer homogenen Paste verknetet. Die Paste wird im folgenden als Katalysatorpaste bezeichnet.

Gleiche Gewichtsteile Basis- und Katalysatorpaste werden durch einen statischen Mischer (Fa. Keller Prozeßtechnik), bestehend aus einem Zylinder mit einer Länge von 60 mm und einem Durchmesser von 5 mm sowie einem statischen Mischer mit 13 Mischelementen, gepresst und das Vermischungsergebnis beurteilt. Die Ergebnisse sind in der folgenden Tabelle wiedergegeben.

**Tabelle**

| Versuchs-Nr. | Vermischungsadditiv [Gew.-% in Katalysatorpaste] | Vermischungsqualität | Lagerstabilität |
|---|---|---|---|
| 1 (Vergleich) | --- | stark marmoriert, teilweise kein Verbund in den unterschiedlichen Schichten | stabil |
| 2 (Vergleich) | Polyethylenoxid (OH-Endgruppen) (Fa. Fluka, Molekulargewicht 2.000) [25 Gew.-%] | deutlich marmoriert teilweise kein Verbund in den unterschiedlichen Schichten | separiert unter Druck |
| 3 (erfindungsgemäss) | Polyalkylenoxidbisbutylurethan (Herstellungsbeispiel 1) [25 Gew.-%] | gut, keine trennbaren Schichten im abgebundenen Material | sehr gut |
| 4 (erfindungsgemäss) | Polyalkylenoxidbiscyclohexylurethan (Herstellungsbeispiel 2) [20 Gew.-%] | gut, keine trennbaren Schichten im abgebundenen Material | sehr gut |
| 5 (erfindungsgemäss) | Polyalkylenoxidbisbutyrat (Herstellungsbeispiel 3) [25 Gew.-%] | gut, keine trennbaren Schichten im abgebundenen Material | sehr gut |
| 6 (erfindungsgemäss) | Polyalkylenoxidbisacetat (Herstellungsbeispiel 4) [30 Gew.-%] | gut, keine trennbaren Schichten im abgebundenen Material | sehr gut |
| 7 (erfindungsgemäss) | Polyethylenoxiddimethylether (Fa. Fluka, Molekulargewicht 2.000) [25 Gew.-%] | schwach marmoriert keine trennbaren Schichten im abgebundenen Material | sehr gut |

### Kommentar

Die Versuche zeigen, dass die erfindungsgemässen Massen hervorragend lagerstabil sind und sich auch unter erhöhtem Druck nicht entmischen. Bei der Vermischung mit einem statischen Mischer erhält man gute Vermischungsergebnisse. Man kann keine Schichten ausmachen, die sich voneinander trennen lassen, im Gegensatz zu den Vergleichsversuchen. Die Vermischungsprodukte der Versuchsnummern 3 bis 7 erfüllen alle Anforderungen, die an eine dentale Abformmasse nach ADA 19 (Prüfung von elastomeren Abformmassen) gestellt werden.

## Patentansprüche

1. Polyetherabformmasse, enthaltend
(a) Polyether,
(b) Katalysator,
(c) Vermischungsadditiv, und
(d) gegebenenfalls übliche Zusatzstoffe,
wobei die Komponenten (a) und (b) räumlich getrennt voneinander vorliegen, dadurch gekennzeichnet, dass das Vermischungsadditiv (c) ein Polyalkylenoxidderivat mit einem Molekulargewicht über 300 ist, welches gegenüber den anderen vorliegenden Komponenten chemisch inert ist und insbesondere keine Hydroxylgruppen, Säuregruppen, primäre Aminogruppen, ionische Gruppen, Halogengruppen oder polymerisationsfähige Gruppen enthält.

2. Polyetherabformmasse nach Anspruch 1, dadurch gekennzeichnet, dass die Komponenten (b), (c) und gegebenenfalls (d) als einheitliche Mischung vorliegen.

3. Polyetherabformmasse nach den Ansprüchen 1 oder 2, dadurch gekennzeichnet, dass das Polyalkylenoxidderivat die folgende allgemeine Formel hat:
R-X-[(CH₂)ₙ-(CHR′)-O]ₖ-[(CH₂)ₘ-(CHR˝)-O]ₗ-(CH₂)ₘ-(CHR˝)-X-R (I)
worin bedeuten:
n = 1 bis 6, vorzugsweise 1 bis 4, insbesondere 1, wobei n innerhalb der Kette variieren kann,
m = 1 bis 6, vorzugsweise 1 bis 4, insbesondere 3, wobei m innerhalb der Kette variieren kann,
k, l = 2 bis 500, bevorzugt 4 bis 250, insbesondere 10 bis 200,
R′, R˝ = H, Methyl, Ethyl, bevorzugt ist R′ = R˝ = H
X = S, O, NH, vorzugsweise O,
R = C₁₋₁₈-, vorzugsweise C₁₋₁₂-, insbesondere C₁₋₆-Alkyl, oder Carbonyl-C₁₋₁₇-, vorzugsweise Carbonyl-C₁₋₁₁-, insbesondere Carbonyl-C₁₋₅-Alkyl, oder einen Rest der allgemeinen Formel II: 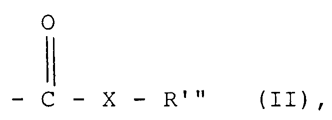
R‴ = C₁₋₁₈-, vorzugsweise C₁₋₁₂-, besonders bevorzugt C₁₋₆-Alkyl und/oder -Aryl bedeutet und X die oben angegebene Bedeutung hat,
wobei die mit den Symbolen k und l indizierten Klammerausdrücke regelmässig oder unregelmässig alternierend oder in Blockform angeordnet sein können.

4. Polyetherabformmasse nach mindestens einem der Ansprüche 1 bis 3, dadurch gekennzeichnet, dass das Polyalkylenoxidderivat ein mittleres Molekulargewicht zwischen 300 und 20000, vorzugsweise zwischen 500 und 10000 aufweist.

5. Polyetherabformmasse nach mindestens einem der Ansprüche 1 bis 4, dadurch gekennzeichnet, dass der Polyether (a) ein aziridinhaltiges Polyalkylenoxid ist.

6. Polyetherabformmasse nach mindestens einem der Ansprüche 1 bis 5, dadurch gekennzeichnet, dass als Katalysator (b) ein Sulfoniumsalz eingesetzt wird.

7. Polyetherabformmasse nach mindestens einem der Ansprüche 1 bis 6, dadurch gekennzeichnet, dass der Polyether (a) in einer Menge von 20 bis 80 Gew.-%, vorzugsweise 32 bis 60 Gew.-%, insbesondere 45 bis 55 Gew.-%, bezogen auf das Gewicht der Gesamtmasse, vorliegt.

8. Polyetherabformmasse nach mindestens einem der Ansprüche 1 bis 7, dadurch gekennzeichnet, dass der Katalysator (b) in einer Menge von 0,2 bis 24 Gew.-%, vorzugsweise 1 bis 18 Gew.-%, insbesondere 5 bis 15 Gew.-%, bezogen auf das Gewicht der Gesamtmasse, vorliegt.

9. Polyetherabformmasse nach mindestens einem der Ansprüche 1 bis 8, dadurch gekennzeichnet, dass das Polyalkylenoxidderivat (c) in einer Menge von 2 bis 60 Gew.-%, vorzugsweise 8 bis 50 Gew.-%, insbesondere 10 bis 40 Gew.-%, bezogen auf das Gewicht der Gesamtmasse, vorliegt.

10. Verwendung eines Polyalkylenoxidderivates als Vermischungsadditiv in Polyetherabformmassen, welches ein Molekulargewicht über 300 aufweist und gegenüber den anderen vorliegenden Komponenten chemisch inert ist und insbesondere keine Hydroxylgruppen, Säuregruppen, primäre Aminogruppen, ionische Gruppen, Halogengruppen oder polymerisationsfähige Gruppen enthält.

11. Verfahren zum Vermischen der Komponenten der Polyetherabformmasse nach mindestens einem der Ansprüche 1 bis 10, wobei als Mischvorrichtung ein statischer Mischer verwendet wird.

## Claims

1. Polyether impression material containing
(a) polyether
(b) catalyst
(c) mixing additive and
(d) optionally the usual additives,
in which the components (a) and (b) are spatially separate from each other, which is characterized in that the mixing additive (c) is a polyalkylene oxide derivative with a molecular weight over 300, which is chemically inert vis-a-vis the other components present and in particular contains no hydroxyl groups, acid groups, primary amino groups, ionic groups, halogen groups or groups capable of polymerization.

2. Polyether impression material according to claim 1, characterized in that the components (b), (c) and, where appropriate, (d) exist as a uniform mixture.

3. Polyether impression material according to claim 1 or 2, characterized in that the polyalkylene oxide derivative has the following general formula:
R-X-[(CH₂)ₙ-(CHR')-O]ₖ-[(CH₂)ₘ-(CHR'')-O]ₗ-(CH₂)ₘ-(CHR'')-X-R (I)
in which:
n = 1 to 6, preferably 1 to 4, particularly 1, such that n can vary within the chain,
m = 1 to 6, preferably 1 to 4, particularly 3, such that m can vary within the chain,
k,l = 2 to 500, preferably 4 to 250, particularly 10 to 200,
R',R'' = H, methyl, ethyl, preferably R' = R'' = H
X = S, O, NH, preferably O,
R = C₁₋₁₈-, preferably C₁₋₁₂-, particularly C₁₋₆-alkyl, or carbonyl-C₁₋₁₇-, preferably carbonyl-C₁₋₁₁-, particularly carbonyl-C₁₋₅-alkyl, or
a radical of the general formula II: 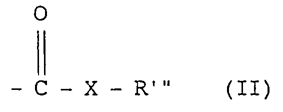 in which
R''' = C₁₋₁₈-, preferably C₁₋₁₂-, particularly preferably C₁₋₆-alkyl and/or -aryl and X has the meaning indicated above,
in which the bracketed expressions indexed with the symbols k and l can be arranged regularly or irregularly alternating or in block form.

4. Polyether impression material according to at least one of the claims 1 to 3, characterized in that the polyalkylene oxide derivative has an average molecular weight between 300 and 20000, preferably between 500 and 10000.

5. Polyether impression material according to at least one of claims 1 to 4, characterized in that the polyether (a) is an aziridine-containing polyalkylene oxide.

6. Polyether impression material according to at least one of claims 1 to 5, characterized in that a sulphonium salt is used as catalyst (b).

7. Polyether impression material according to at least one of claims 1 to 6, characterized in that the polyether (a) is present in a quantity of 20 to 80 % by weight, preferably 32 to 60 % by weight, particularly 45 to 55 % by weight, relative to the weight of the total mass.

8. Polyether impression material according to at least one of claims 1 to 7, characterized in that the catalyst (b) is present in a quantity of 0.2 to 24 % by weight, preferably 1 to 18 % by weight, particularly 5 to 15 % by weight, relative to the weight of the total mass.

9. Polyether impression material according to at least one of claims 1 to 8, characterized in that the polyalkylene oxide derivative (c) is present in a quantity of 2 to 60 % by weight, preferably 8 to 50 % by weight, particularly 10 to 40 % by weight, relative to the weight of the total mass.

10. Use of a polyalkylene oxide derivative as a mixing additive in polyether impression materials, which has a molecular weight above 300 and is chemically inert vis-a-vis the other components present and in particular does not contain hydroxyl groups, acid groups, primary amino groups, ionic groups, halogen groups or groups capable of polymerization.

11. Process for mixing the components of the polyether impression material according to at least one of claims 1 to 10, in which a static mixer is used as the mixing device.

## Revendications

1. Mélange à mouler à base d'un polyéther, contenant :
(a) un polyéther,
(b) un catalyseur,
(c) un additif de mélange, et
(d) éventuellement des additifs usuels
les constituants (a) et (b) étant spatialement séparés l'un de l'autre, caractérisé en ce que l'additif de mélange (c) est un dérivé d'un poly(oxyde d'alkylène) ayant une masse moléculaire supérieure à 300, chimiquement inerte vis-à-vis des autres constituants présents, et ne contenant en particulier pas de groupes hydroxyle, de groupes acides, de groupes amino primaires, de groupes ioniques, de groupes halogéno ou de groupes polymérisables.

2. Mélange à mouler à base d'un polyéther selon la revendication 1, caractérisé en ce que les constituants (b), (c) et éventuellement (d) se présentent sous forme d'un mélange homogène.

3. Mélange à mouler à base d'un polyéther selon les revendications 1 ou 2, caractérisé en ce que le dérivé de poly(oxyde d'alkylène) a la formule générale suivante :
R-X-[(CH₂)ₙ-(CHR')-O]ₖ-[(CH₂)ₘ-(CHR'')-O]ₗ-(CH₂)ₘ-(CHR'')-X-R (I)
dans laquelle
n = 1 à 6, de préférence 1 à 4, en particulier 1, n pouvant varier le long de la chaîne,
m = 1 à 6, de préférence 1 à 4, en particulier 3, m pouvant varier le long de la chaîne,
k, l = 2 à 500, de préférence 4 à 250, en particulier 10 à 200,
R' et R'' sont des hydrogènes ou des radicaux méthyle ou éthyle, et de préférence R' = R'' = H,
X = S, O, NH, de préférence O,
R est un radical alkyle en C₁-C₁₈, de préférence en C₁-C₁₂ et en particulier en C₁-C₆, ou encore un radical carbonyl-(alkyle en C₁-C₁₇, de préférence en C₁-C₁₁, en particulier en C₁-C₅), ou encore un radical de formule générale II : 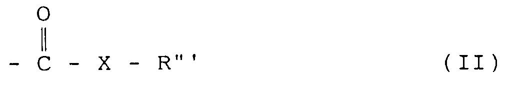 dans laquelle
R''' est un radical alkyle et/ou aryle en C₁-C₁₈, de préférence en C₁-C₁₂ et tout particulièrement en C₁-C₆, et X a les significations données ci-dessus,
les expressions entre crochets, portant les indices k et l, pouvant être disposées en alternance régulière ou irrégulière, ou sous forme de séquences.

4. Mélange à mouler à base d'un polyéther selon au moins l'une des revendications 1 à 3, caractérisé en ce que le dérivé de poly(oxyde d'alkylène) a une masse moléculaire moyenne de 300 à 20 000 et de préférence de 500 à 10 000.

5. Mélange à mouler à base d'un polyéther selon au moins l'une des revendications 1 à 4, caractérisé en ce que le polyéther (a) est un poly(oxyde d'alkylène) contenant des groupes aziridine.

6. Mélange à mouler à base d'un polyéther selon au moins l'une des revendications 1 à 5, caractérisé en ce qu'on utilise comme catalyseur (b) un sel de sulfonium.

7. Mélange à mouler à base d'un polyéther selon au moins l'une des revendications 1 à 6, caractérisé en ce que le polyéther (a) est présent en une quantité, par rapport au poids de la composition totale, de 20 à 80, de préférence de 32 à 60 et en particulier de 45 à 55 % en poids.

8. Mélange à mouler à base d'un polyéther selon au moins l'une des revendications 1 à 7, caractérisé en ce que le catalyseur (b) est présent en une quantité, par rapport au poids de la composition totale, de 0,2 à 24, de préférence de 1 à 18 et en particulier de 5 à 15 % en poids.

9. Mélange à mouler à base d'un polyéther selon au moins l'une des revendications 1 à 8, caractérisé en ce que le dérivé de poly(oxyde d'alkylène) (c) est présent en une quantité, par rapport au poids de la composition totale, de 2 à 60, de préférence de 8 à 50 et en particulier de 10 à 40 % en poids.

10. Utilisation d'un dérivé de poly(oxyde d'alkylène) comme additif de mélange dans des mélanges à mouler à base d'un polyéther, qui a une masse moléculaire supérieure à 300 et est chimiquement inerte vis-à-vis des autres constituants présents, et en particulier ne contient pas de groupes hydroxyle, de groupes acides, de groupes amino primaire, de groupes ioniques, de groupes halogéno ou de groupes polymérisables.

11. Procédé pour mélanger les constituants du mélange à mouler à base d'un polyéther selon au moins l'une des revendications 1 à 10, dans lequel on utilise comme dispositif de mélange un mélangeur statique.
